# EUROPEAN PATENT SPECIFICATION

(11) **EP 0 461 343 B1**
(45) Date of publication and mention of the grant of the patent: **19.06.1996**
(21) Application number: 91103016.1
(22) Date of filing: 28.02.1991
(51) Int. Cl.: C07D 311/94, A61K 31/35

(54) **Cyclopropachromen derivatives**
Cyclopropachromenderivate
Dérivés de cyclopropachromène

(30) Priority: 06.06.1990 JP 147710/90
(43) Date of publication of application: 18.12.1991
(73) Proprietor: SUNTORY LIMITED, Kita-ku, Osaka-shi, Osaka 530 (JP)
(72) Inventor: Tatsuoka, Toshio, Nishinomiya-shi, Hyogo-ken (JP); Nomura, Kayoko, Takatsuki-shi, Osaka (JP)
(74) Representative: Hansen, Bernd, Dr. Dipl.-Chem.

(56) References cited:
- EP-A- 0 238 883

## Description

This invention relates to a novel cyclopropachromen derivative represented by formula (I) shown below and a pharmaceutically acceptable salt thereof.

The compounds according to the present invention have a wide use as improving and treating agents for cerebral organic and functional disorders.

Living cerebral cells maintain their intracellular environment quite differently from the surrounding environment (extracellular fluid). Maintenance of such a difference requires constant production and supply of energy. Most of the energy required for cerebral nerve cells is furnished by oxygen and glucose. These energy sources, not substantially stored in the brain, must always be supplied from blood.

Should some disturbance take place in the brain resulting in a cut off of the supply of oxygen and glucose, it generally follows that an metabolic energy deficiency would stepwise proceed, and the cells would gradually lose their functions over time and eventually undergo structural disintegration and fail to perform their normal functions.

Hence, the brain has a well-developed mechanism for conditioning the blood flow of its own blood vessels in order to stably supply energy sources to the cerebral tissue and to maintain the extracellular environmental conditions constant.

For internal treatments of disturbances of cerebral blood vessels, various kinds of drugs such as cerebral circulation improving agents, cerebral vasodilators, cerebral metabolism improving agents, and the like have been made use of. Under the present circumstances, however, these drugs bring about no substantial alleviation in neurotic or psychic symptoms, though alleviation in subjective symptoms are obtained.

EP-A-0238883 discloses various oxabicycloheptane derivatives which are pharmacologically useful for ameliorating or eliminating the symptoms that appear either as a result of organic disorders in the brain or on account of pathergasia.

The inventors have conducted extensive and intensive studys on a compound effective for improving and treating symptoms arising from various disturbances in the brain. As a result, it has now been found that the novel cyclopropachromen derivatives and their salts according to the present invention are extremely effective for treatment of various oxygen deficit conditions (cerebral anoxia) in cerebral nerve cells which are believed to be closely related to the above-mentioned disturbances, thus completing the present invention.

The novel cyclopropachromen derivatives of the present invention exhibit cerebral function improving activities in experimental model animals suffering from various cerebral anoxemic conditions even at low dose levels and are, therefore, effective in alleviating and treating organic and functional disorders.

The present invention provides a cyclopropachromen derivative represented by formula (I): 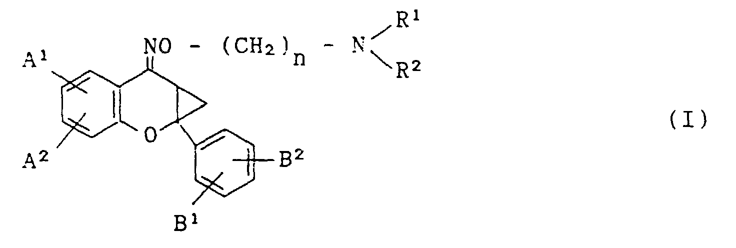 wherein n represents an integer of 2 to 5; R¹ and R² independently represent a hydrogen atom or an alkyl group having from 1 to 5 carbon atoms, a phenyl group or an aralkyl group having 7 to 10 carbon atoms;
A¹ and A² independently represent a hydrogen atom, a hydroxyl group, a halogen atom or a
C₁₋₁₀ alkoxy group optionally substituted by a phenyl group or a pyridyl group; B¹ and B² independently represent a hydrogen atom, a hydroxyl group, a halogen atom or a
C₁₋₁₀ alkoxy group optionally substituted by a phenyl group or a pyridyl group; with the proviso that both substituents or at least one of the benzene rings represent substituents other than hydrogen atoms, and a pharmaceutically acceptable salt thereof.

The term "halogen" as used herein includes fluoro, chloro, bromo or iodo, and fluoro, chloro and bromo are preferred.

The integer represented by n is preferably 2 - 4 and more preferably 2 or 3.

Examples of the alkyl group represented by R¹ and R² may be straight or branched. Those having 1 - 3 carbon atoms such as methyl and ethyl are preferred.

Examples of the aralkyl group represented by R¹ and R² include benzyl and phenethyl.

It should be understood that the compound of the present invention comprises several isomers as schematically illustrated below. Namely, there are two geometrical isomers (E-form and Z-form) at the oxime structure, and further, each of the geometrical isomers has two optical isomers. Each isomers can be separated by a conventional manner by way of, e.g. recrystallization, column chromatography, TLC, HPLC or by using a chemical substance commonly used in the separation of optical isomers.

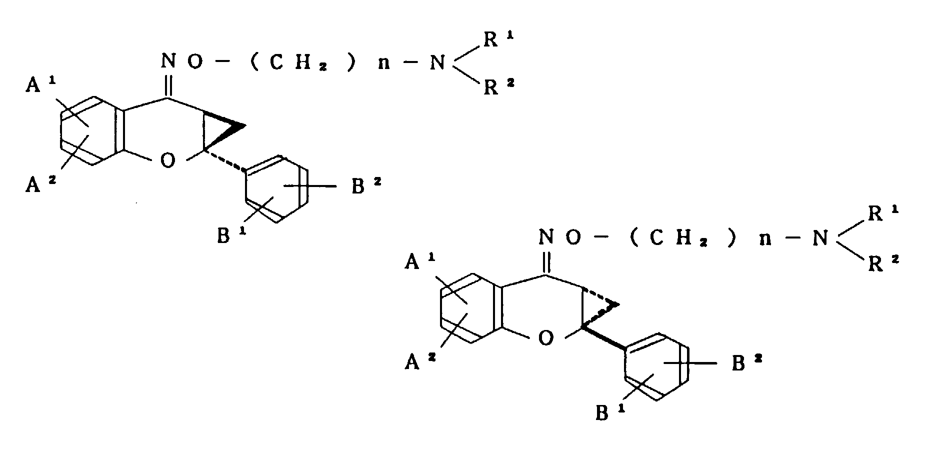

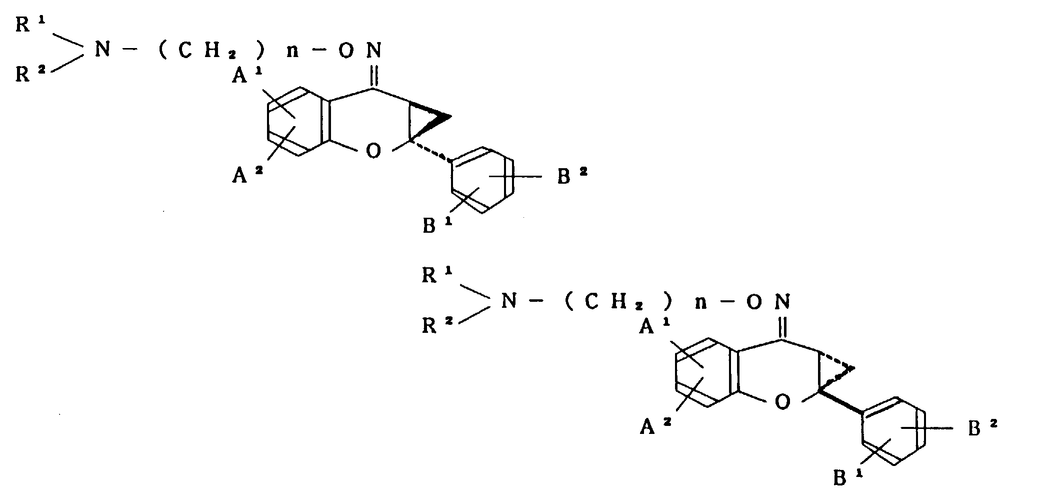

The compounds represented by formula (I) can be synthesized in accordance with the process described in Japanese Patent Application Laid-Open No. Sho-62-198676 as illustrated by the following reaction scheme:

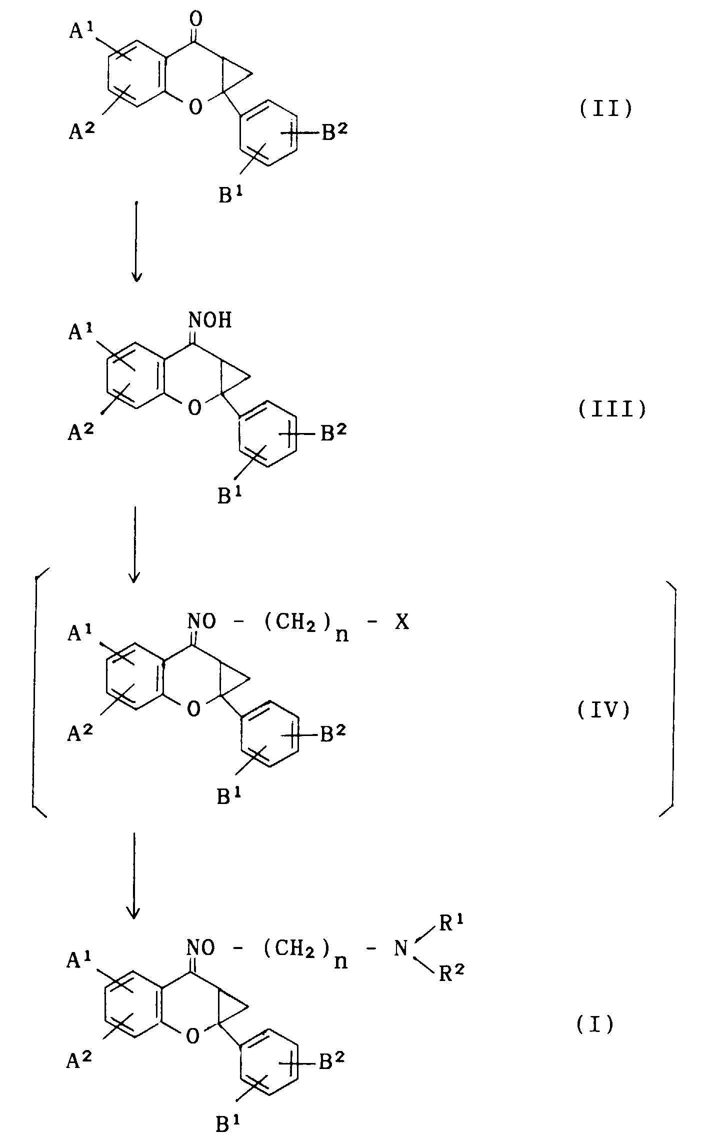

Compound (II) is reacted with hydroxylamine hydrochloride in pyridine to obtain compound (III). Compound (III) is then condensed with a halogenated amine compound of formula: 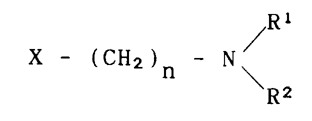 wherein R¹, R² and n are as defined above; and X represents a halogen atom,
to obtain compound (I).

Alternatively, compound (III) is condensed with a bifunctional compound represented by formula:

X¹ - (CH₂)ₙ - X²

wherein X¹ represents a halogen atom; and X² represents a halogen atom or an ethylene oxide group,
to form compound (IV), which is then reacted with an appropriate amine compound to obtain compound (I).

The starting compound (II) is known per se as disclosed in P. Bennett, et al., J. Chem. Soc., Perkin Trans., I, No. 12, p. 2990 (1979) or can be synthesized by the process disclosed therein.

The compounds according to the present invention have low toxicity and can be formulated either as such or as a salt thereof, such as inorganic acid salts (e.g., hydrochloride, sulfate, nitrate, phosphate), organic acid salts (e.g., acetate, propionate, butyrate, tartrate, malonate, succinate, maleate, fumarate, oxalate, citrate, malate, p-toluenesulfonate, methanesulfonate), and alkali metal salts (e.g., sodium salt, potassium salt) in cases where either A¹ or A² is a hydroxyl group, together with known carriers into various preparations for the improvement and treatment of symptoms caused by various disturbances in the brain. For example, the active ingredient is formulated either alone or in combination with commonly employed vehicles, etc. into appropriate dosage forms for oral or non-oral administration, such as capsules, tablets, injectable solutions, etc.

These preparations can be prepared, for example, as follows. Capsules are prepared by mixing a powdered active ingredient with vehicles, e.g., lactose, starch or a derivative thereof, a cellulose derivative, etc., and charging the mixture in gelatin capsules. Tablets are prepared by mixing the active ingredient with the above-mentioned vehicles and, in addition, binders, e.g., sodium carboxymethyl cellulose, alginic acid, gum arabic, etc., and water, granulating the mixture if desired, adding lubricants, e.g., talc, stearic acid, etc., to the mixture, and punching the mixture by means of a conventional compressive punching machine. Injectable solutions for non-oral administration are prepared by dissolving the active ingredient in sterilized distilled water or sterilized physiological saline together with dissolving aids and sealing the solution into ampules. If desired, the injectable solutions may contain stabilizers, buffering agents, and so on.

Synthesis of the compounds according to the present invention will be illustrated below by way of Reference Examples and Examples. Reference Examples relate to preparation of starting compounds to be used in the synthesis of the compounds of formula (I). Reference Examples are referred to with a combination of two numbers, in which the first number corresponds to the above-mentioned formula (II) to (IV) [e.g., Reference Example II-1 relates to preparation of compound (II)].

### Reference Example II-1

### 1a,7a-dihydro-4,5-dimethoxy-1a-phenylcyclopropa[b]chromen-7-(1H)-one

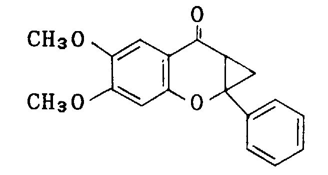

In 100 mℓ of dimethyl sulfoxide was dissolved 6.78 g (30.8 mmol) of trimethylsulfoxonium iodide, and 1.24 g (30.8 mmol) of sodium hydride (60% oil dispersion) was added to the solution in small portions. The mixture was stirred until evolution of hydrogen ceased. A dimethyl sulfoxide solution having dissolved therein 5.82 g (20.5 mmol) of 6,7-dimethoxyflavone was then added to the reaction mixture, followed by stirring at room temperature for 4 hours. The reaction mixture was poured into ice-water and extracted with diethyl ether. The extract was washed with water and dried over anhydrous magnesium sulfate. After filtration, the filtrate was concentrated, and the residue was purified by silica gel column chromatography using a mixed solvent of hexane-ethyl acetate (70:30) as an eluent to obtain 2.72 g (yield: 44.8%) of the titled compound.

### Reference Examples II-2 to II-6

The following compounds were synthesized in the same manner as in Reference Example II-1.
1a,7a-dihydro-3,4-dimethoxy-1a-phenylcylopropa[b]chromen-7(1H)-one (Reference Example II-2);
4,5-dichloro-1a,7a-dihydro-1a-phenylcyclopropa[b]chromen-7(1H)-one (Reference Example II-3);
4,5-dibenzyloxy-1a,7a-dihydro-1a-phenylcylopropa[b]chromen-7(1H)-one (Reference Example II-4);
1a-(3,4-dichlorophenyl)-1a,7a-dihydrocyclopropa[b]chromen-7(1H)-one (Reference Example II-5);
1a,7a-dihydro-1a-(3,4-dimethoxyphenyl)cyclopropa[b]chromen-7(1H)-one (Reference Example II-6);

Physical properties of the compounds obtained in Reference Examples II-1 through II-6 are shown in Table 1 below.

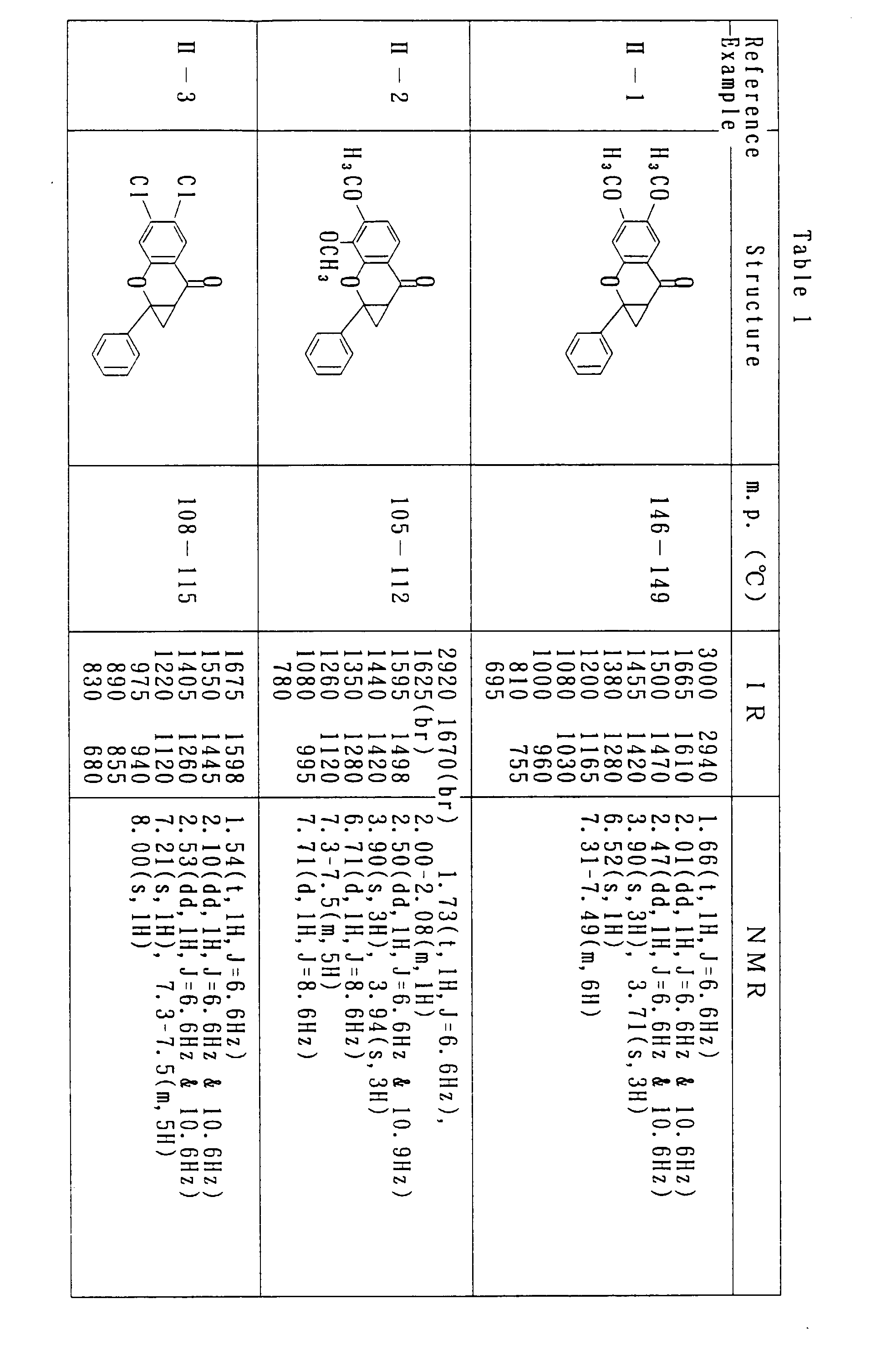

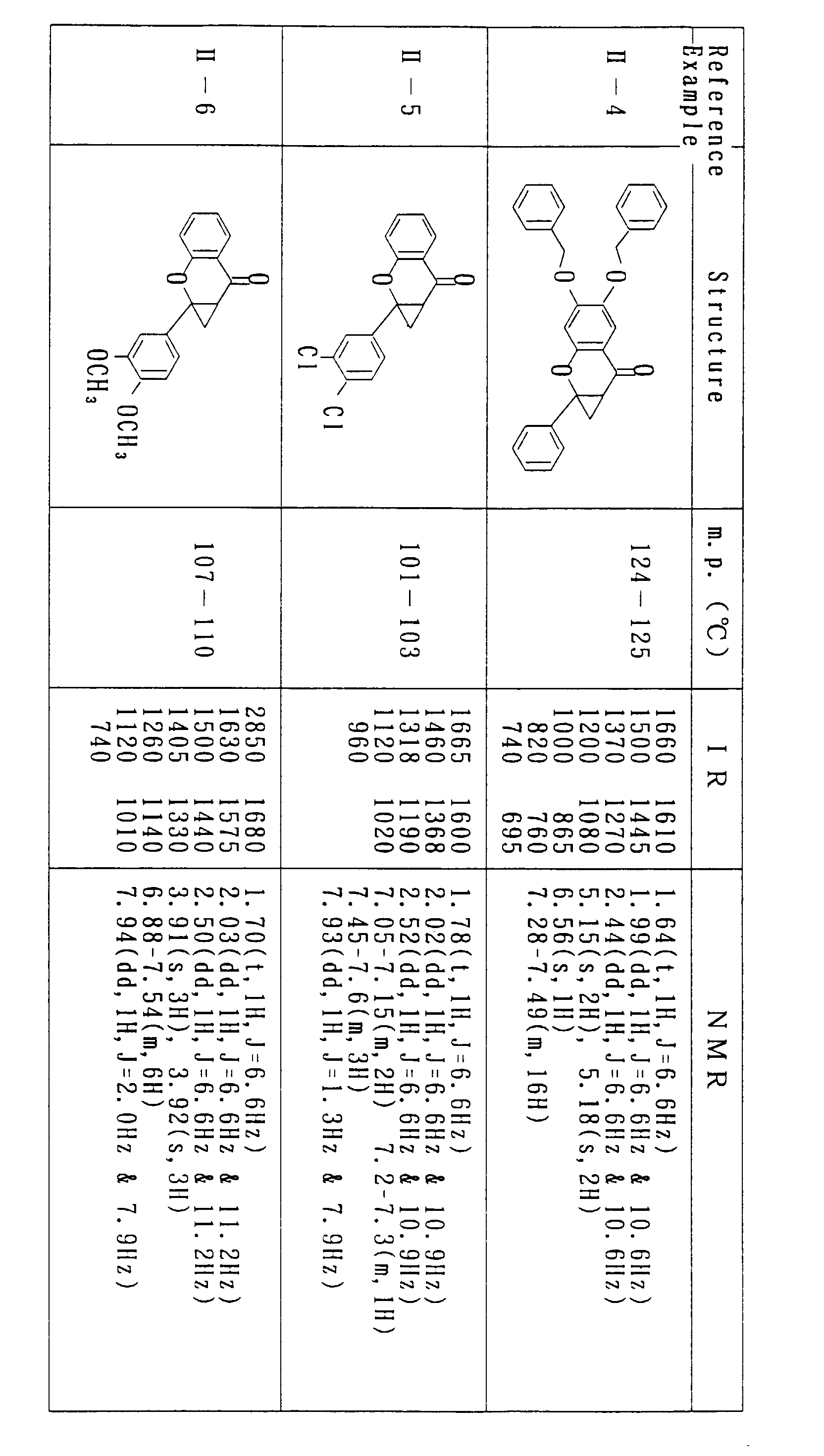

### Reference Example III-1

### 1a,7a-dihydro-4,5-dimethoxy-7(1H)-hydroxyimino-1a-phenylcyclopropa[b]chromen

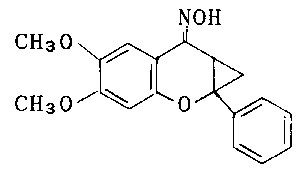

In 50 mℓ of pyridine was dissolved 500 mg (1.69 mmol) of the compound obtained in Reference Example II-1, and 469 mg (6.76 mmol) of hydroxylamine hydrochloride was added thereto, followed by stirring at 100°C for 1 hour. The reaction mixture was concentrated, diluted with water, and extracted with chloroform. The chloroform extract was washed with water and dried over anhydrous magnesium sulfate. After filtration, the filtrate was concentrated, and the residue was purified by silica gel column chromatography using a mixed solvent of hexane-ethyl acetate (2:1 by volume) as an eluent to obtain 492 mg (93.6%) of the titled compound.

### Reference Examples III-2 to III-6

The following compounds were synthesized in the same manner as in Reference Example III-1.
1a,7a-dihydro-3,4-dimethoxy-7(1H)-hydroxyimino-1a-phenylcyclopropa[b]chromen (Reference Example III-2);
4,5-dichloro-1a,7a-dihydro-7(1H)-hydroxyimino-1a-phenylcyclopropa[b]chromen (Reference Example III-3);
4,5-dibenzyloxy-1a,7a-dihydro-7(1H)-hydroxyimino-1a-phenylcyclopropa[b]chromen (Reference Example III-4);
1a-(3,4-dichlorophenyl)-1a,7a-dihydro-7(1H)-hydroxyiminocyclopropa[b]chromen (Reference Example III-5);
1a,7a-dihydro-1a-(3,4-dimethoxyphenyl)-7(1H)-hydroxyiminocyclopropa[b]chromen (Reference Example III-6);

Physical properties of the compounds obtained in Reference Examples III-1 through III-6 are shown in Table 2 below.

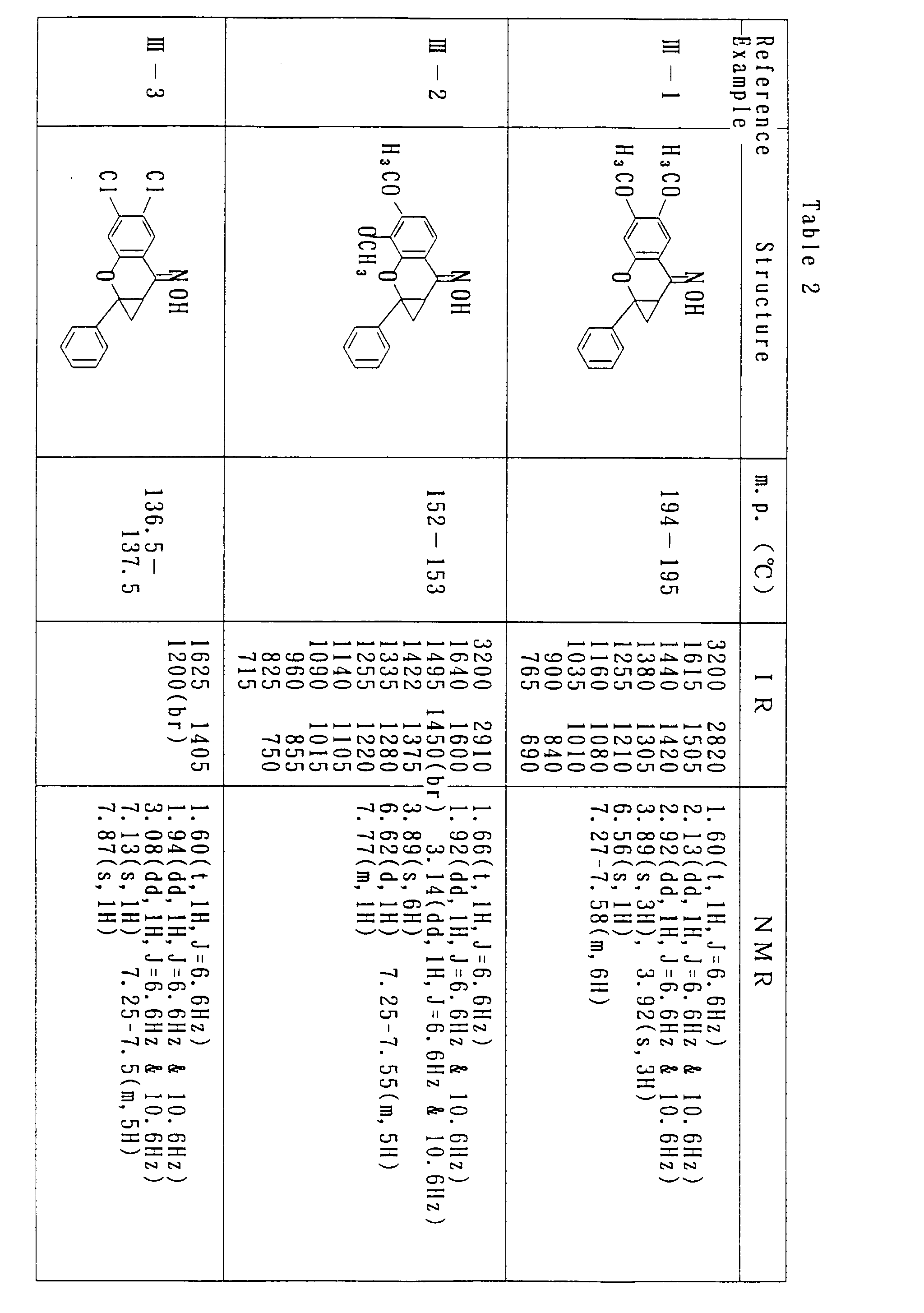

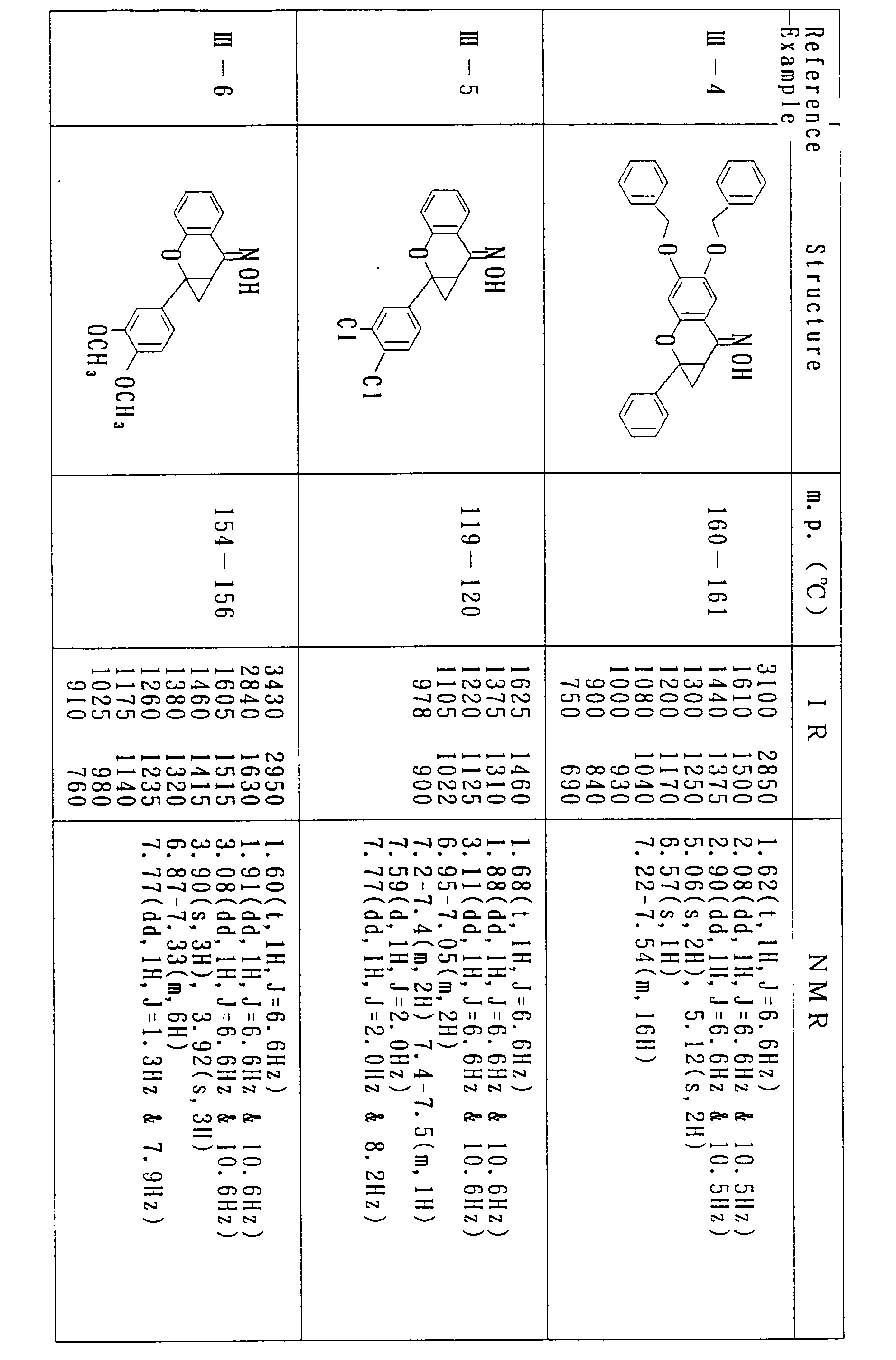

### Reference Example IV-1

### 7(1H)-(2-chloroethyloxyimino)-4,5-dimethoxy-1a,7a-dihydro-1a-phenylcyclopropa[b]chromen

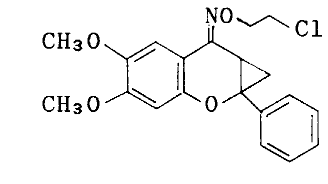

In 20 mℓ of dioxane was dissolved 300 mg of the compound obtained in Reference Example III-1, and 57.9 mg (1.5 equivalents) of sodium hydride (60% oil dispersion) was added to the solution. Then, 0.482 mℓ (6 equivalents) of 1-bromo-2-chloroethane was added thereto, and the reaction mixture was heated at 100°C for 5 hours with stirring, followed by concentration. The residue was diluted with water and extracted with diethyl ether. The diethyl ether layer was washed with water and dried over anhydrous magnesium sulfate. After filtration, the filtrate was concentrated, and the residue was purified by silica gel column chromatography using a mixed solvent of hexane and ethyl acetate (85:15 by volume) as an eluent to obtain 275 mg (76.3%) of the titled compound.

### Reference Example IV-2

### 7(1H)-(3-chloropropyloxyimino)-4,5-dimethoxy-1a,7a-dihydro-1a-phenylcyclopropa[b]chromen

The titled compound was synthesized from the compound of Reference Example III-1 in the same manner as in Reference Example IV-1.

Physical properties of the compounds obtained in Reference Examples IV-1 and IV-2 are shown in Table 3 below.

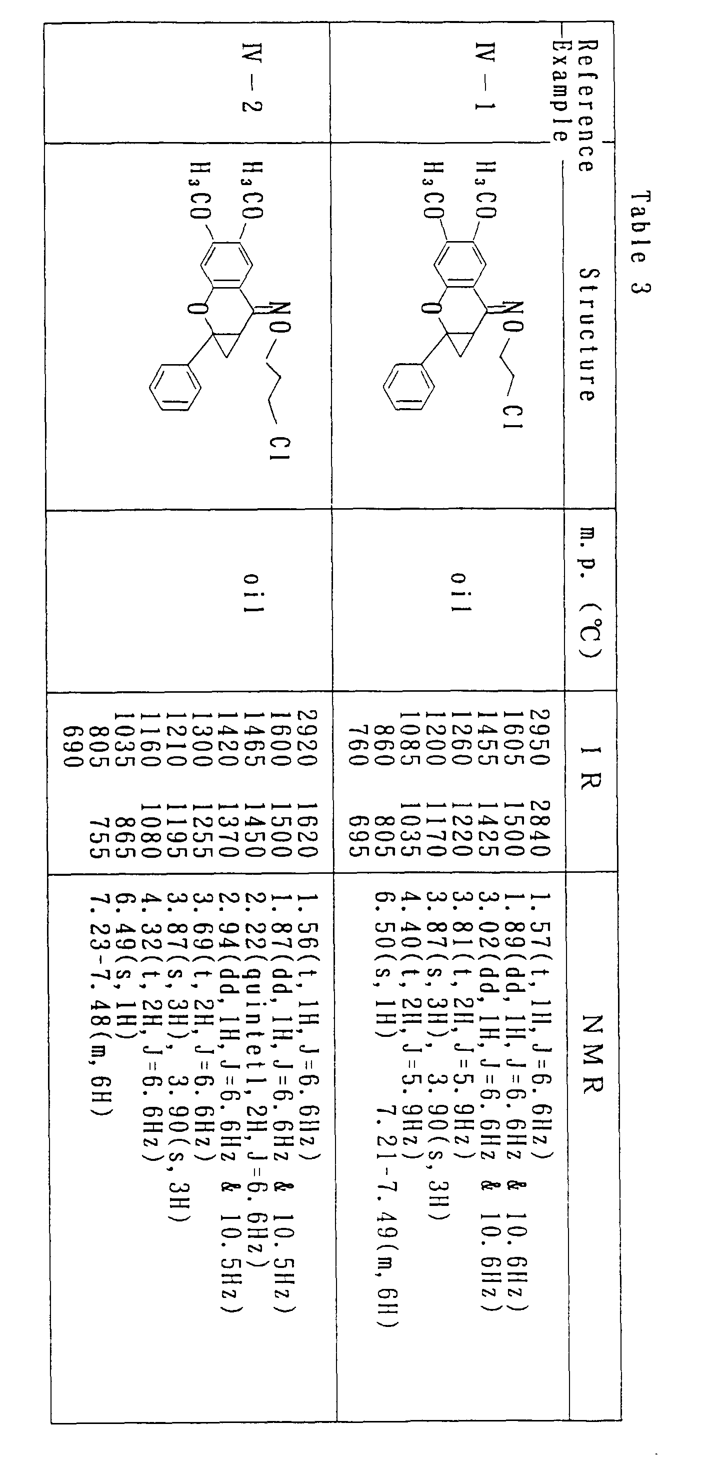

### Example 1

### 1a,7a-dihydro-4,5-dimethoxy-7(1H)-(2-methylaminoethyloxyimino)-1a-phenylcyclopropa[b]chromen

### [Compound of formula (I) wherein A¹ = 4-OCH₃; A² = 5-OCH₃; B¹ = H; B² = H; NR¹R² = NHCH³; and n = 2]

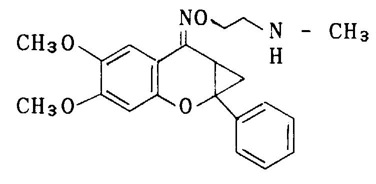

In 10 mℓ of dioxane was dissolved 270 mg of the compound obtained in Reference Example IV-1, and 10 mℓ of a saturated mono-methylamine solution in dioxane was added to the solution. The mixture was heated in a closed tube at 100°C for 17 hours and then freed of dioxane by distillation. Water and a sodium hydroxide aqueous solution were added to the residue, and the residue was extracted with methylene chloride. The extract was washed with water and dried over magnesium sulfate. After filtration, the filtrate was concentrated, and the residue was purified by silica gel column chromatography using a mixed solvent of methylene chloride and methanol (9:1) as an eluent to obtain 165 mg (62.0%) of the titled compound.

The resulting compound was converted to its L-tartrate in a usual manner.

### Example 2

### 4,5-dichloro-1a,7a-dihydro-7(1H)-(2-dimethylaminoethyloxyimino)-1a -phenylcyclopropa[b]chromen [Compound of formula (I) wherein A¹ = 4-Cl; A² = 5-Cl; B¹ = H, B² = H; NR¹R² = N(CH₃)₂; and n = 2]

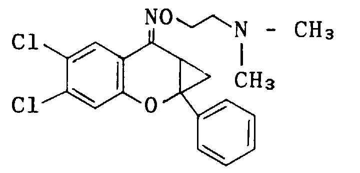

In 7 mℓ of tetrahydrofuran was dissolved 95 mg (0.3 mmol) of the compound obtained in Reference Example III-3, and 18 mg (0.45 mmol) of sodium hydride (60% oil dispersion) was added thereto, followed by stirring at room temperature for 45 minutes. To the reaction mixture was added 128 mg (1.19 mmol) of dimethylaminoethyl chloride, followed by refluxing under heating for 17 hours. The reaction mixture was concentrated, and the residue was diluted with ice-water and extracted with diethyl ether. The extract was washed with water and dried over anhydrous magnesium sulfate. After filtration, the filtrate was concentrated, and the residue was purified by neutral silica gel column chromatography using a mixed solvent of methylene chloride and methanol (95:5) as an eluent to obtain 112 mg (99.0%) of the titled compound.

The resulting compound was converted to its maleate in a usual manner. The physical properties of the maleate are shown in Table 4 below.

### Example 3

### 1a,7a-dihydro-4,5-dimethoxy-7(1H)-(2-dimethylaminoethyloxyimino)-1a -phenylcyclopropa[b]chromen [Compound of formula (I) wherein A¹ = 4-OCH₃; A² = 5-OCH₃; B¹ = H; B² = H; NR¹R² = N(CH₃)₂; n = 2]

The titled compound was synthesized from the compound of Reference Example III-1 in the same manner as in Example 2.

### Example 4

### 1a-(3,4-dichlorophenyl)-1a,7a-dihydro-7(1H)-(2-dimethylaminoethyloxyimino)cyclopropa[b]chromen [Compound of formula (I) wherein A¹ = H; A² = H; B¹ = 3-Cl; B² = 4-Cl; NR¹R² = N(CH₃)₂; n = 2]

The titled compound was synthesized from the compound of Reference Example III-5 in the same manner as in Example 2.

### Example 5

### 7(1H)-2-(diethylaminoethyloxyimino)-1a,7a-dihydro-4,5-dimethoxy-1a-phenylcyclopropa[b]chromen [Compound of formula (I) wherein A¹ = 4-OCH₃; A² = 5-OCH₃; B¹ = H; B² = H; NR¹R² = N(C₂H₅)₂; n = 2]

The titled compound was synthesized from the compound of Reference Example III-1 in the same manner as in Example 2.

### Example 6

### 4,5-dichloro-1a,7a-dihydro-7(1H)-(3-methylaminopropyloxyimino)-1a-phenylcyclopropa[b]chromen [Compound of formula (I) wherein A¹ = 4-Cl; A² = 5-Cl; B¹ = H; B² = H; NR¹R² = NHCH₃; n = 3]

The titled compound was synthesized from the compound of Reference Example III-3 in the same manner as in Example 2.

### Example 7

### 1a,7a-dihydro-4,5-dimethoxy-7(1H)-(3-methylaminopropyloxyimino)-1a-phenylcyclopropa[b]chromen [Compound of formula (I) wherein A¹ = 4-OCH₃; A² = 5-OCH₃; B¹ = H; B² = H; NR¹R² = NHCH₃; n = 3]

The titled compound was synthesized from the compound of Reference Example IV-2 in the same manner as in Example 1.

### Example 8

### 1a-(3,4-dichlorophenyl)-1a,7a-dihydro-7(1H)-(3-methylaminopropyloxyimino)cyclopropa[b]chromen [Compound of formula (I) wherein A¹ = H; A² = H; B¹ = 3-Cl; B² = 4-Cl; NR¹R² = NHCH₃; n = 3]

The titled compound was synthesized from the compound of Reference Example III-5 in the same manner as in Example 2.

### Example 9

### 4,5-dichloro-1a,7a-dihydro-7(1H)-(3-ethylaminopropyloxyimino)-1a-phenylcyclopropa[b]chromen [Compound of formula (I) wherein A¹ = 4-Cl; A² = 5-Cl; B¹ = H; B² = H; NR¹R² = NHC₂H₅; n = 3]

The titled compound was synthesized from the compound of Reference Example III-3 in the same manner as in Example 2.

### Example 10

### 1a-(3,4-dichlorophenyl)-1a,7a-dihydro-7(1H)-3-ethylaminopropyloxyimino)cyclopropa[b]chromen [Compound of formula (I) wherein A¹ = H; A² = H; B¹ = 3-Cl; B² = 4-Cl; NR¹R² = NHC₂H₅; n = 3]

The titled compound was synthesized from the compound of Reference Example III-5 in the same manner as in Example 2.

### Example 11

### 1a,7a-dihydro-7(1H)-(3-dimethylaminopropyloxymino)-3,4-dimethoxy-la-phenylcyclopropa[b]chromen [Compound of formula (I) wherein A¹ = 3-OCH₃; A² = 4-OCH₃; B¹ = H; B² = H; NR¹R² = N(CH₃)₂; n = 3]

The titled compound was synthesized from the compound of Reference Example III-2 in the same manner as in Example 2.

### Example 12

### 4,5-dichloro-1a,7a-dihydro-7(1H)-(3-dimethylaminopropyloxyimino)-1a -phenylcyclopropa[b]chromen [Compound of formula (I) wherein A¹ = 4-Cl; A² = 5-Cl; B¹ = H; B² = H; NR¹R² = N(CH₃)₂; n = 3]

The titled compound was synthesized from the compound of Reference Example III-3 in the same manner as in Example 2.

### Example 13

### 1a,7a-dihydro-4,5-dimethoxy-7(1H)-(3-dimethylaminopropyloxyimino)-1a -phenylcyclopropa[b]chromen [Compound of formula (I) wherein A¹ = 4-OCH₃; A² = 5-OCH₃; B¹ = H; B² = H; NR¹R² = N(CH₃)₂; n = 3]

The titled compound was synthesized from the compound of Reference Example III-I in the same manner as in Example 2.

### Example 14

### 4,5-dibenzyloxy-1a,7a-dihydro-7(1H)-(3-dimethylaminopropyloxyimino)cyclopropa[b]chromen [Compound of formula (I) wherein A¹ = 4-OCH₂-C₆H₅; A² = 5-OCH₂-C₆H₅; B¹ = H; B² = H; NR¹R² = N(CH₃)₂; n = 3]

The titled compound was synthesized from the compound of Reference Example III-4 in the same manner as in Example 2.

### Example 15

### 1a-(3,4-dichlorophenyl)-1a,7a-dihydro-7(1H)-(3-dimethylaminopropyloxyimino)cyclopropa[b]chromen [Compound of formula (I) wherein A¹ = H; A² = H; B¹ = 3-Cl; B² = 4-Cl; NR¹R² = N(CH₃)₂; n = 3]

The titled compound was synthesized from the compound of Reference Example III-5 in the same manner as in Example 2.

### Example 16

### 1a,7a-dihydro-7(1H)-(3-dimethylaminopropyloxyimino)-1a-(3,4-dimethoxyphenyl)cyclopropa[b]chromen [Compound of formula (I) wherein A¹ = H; A² = H; B¹ = 3-OCH₃; B² = 4-OCH₃; NR¹R² = N(CH₃)₂; n = 3]

The titled compound was synthesized from the compound of Reference Example III-6 in the same manner as in Example 2.

### Example 17

### 1a,7a-dihydro-4,5-dihydroxy-7(1H)-(3-dimethylaminopropyloxyimino)cyclopropa[b]chromen [Compound of formula (I) wherein A¹ = 4-OH; A² = 5-OH; B¹ = H; B² = H; NR¹R² = N(CH₃)₂; n = 3]

In 20 mℓ of ethyl acetate was suspended 48 mg of 10% palladium-on-carbon. After displacing the atmosphere with hydrogen by suction, 240 mg of the compound obtained in Example 14 was added to the suspension, and the mixture was stirred at room temperature for 5 hours in a hydrogen stream (atmospheric pressure). The reaction mixture was worked-up in the same manner as in Example 2 to obtain 67.0 mg (41.5%) of the titled compound.

Physical properties of the compounds obtained in Examples 1 to 17 are shown in Table 4 below.

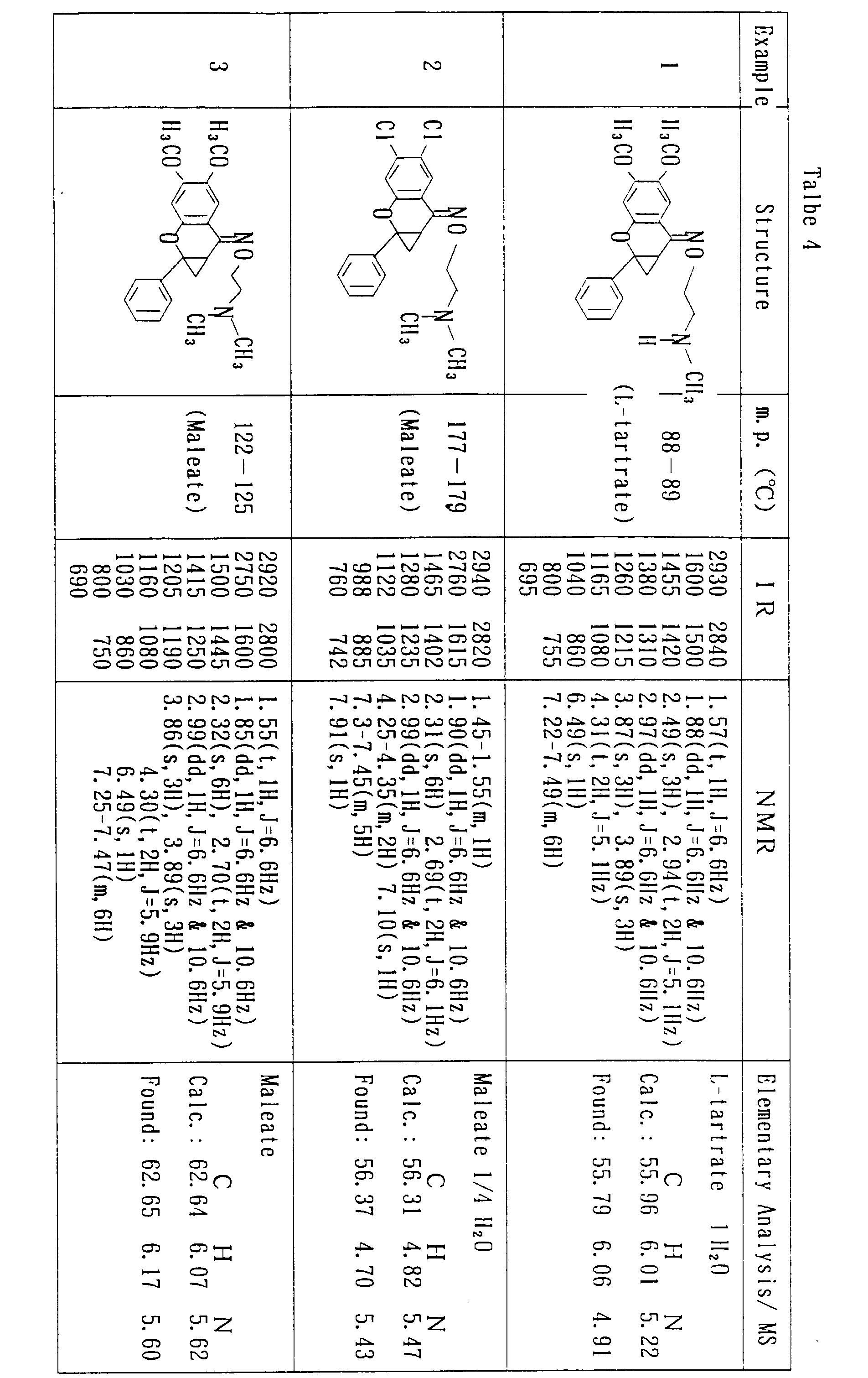

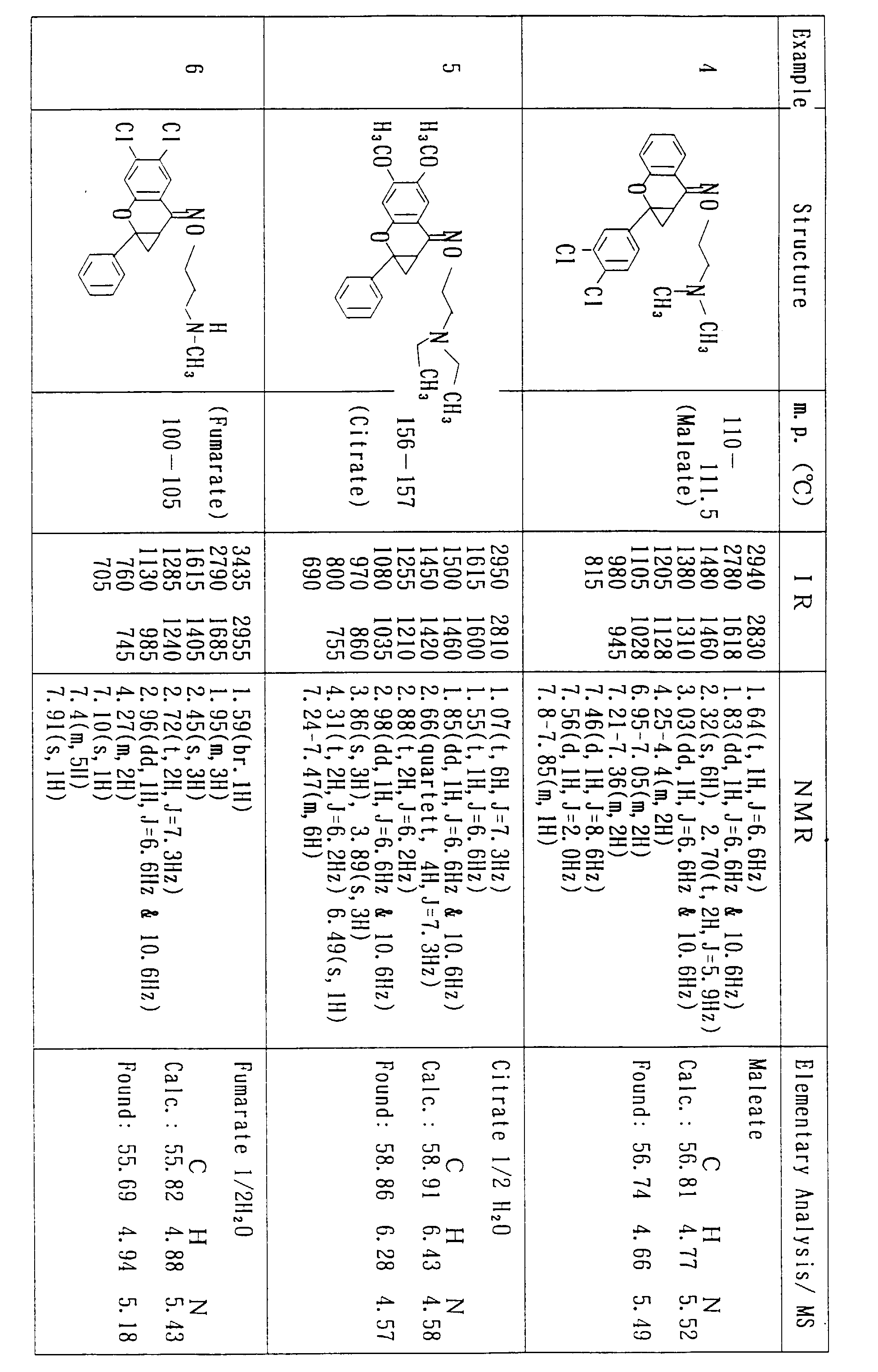

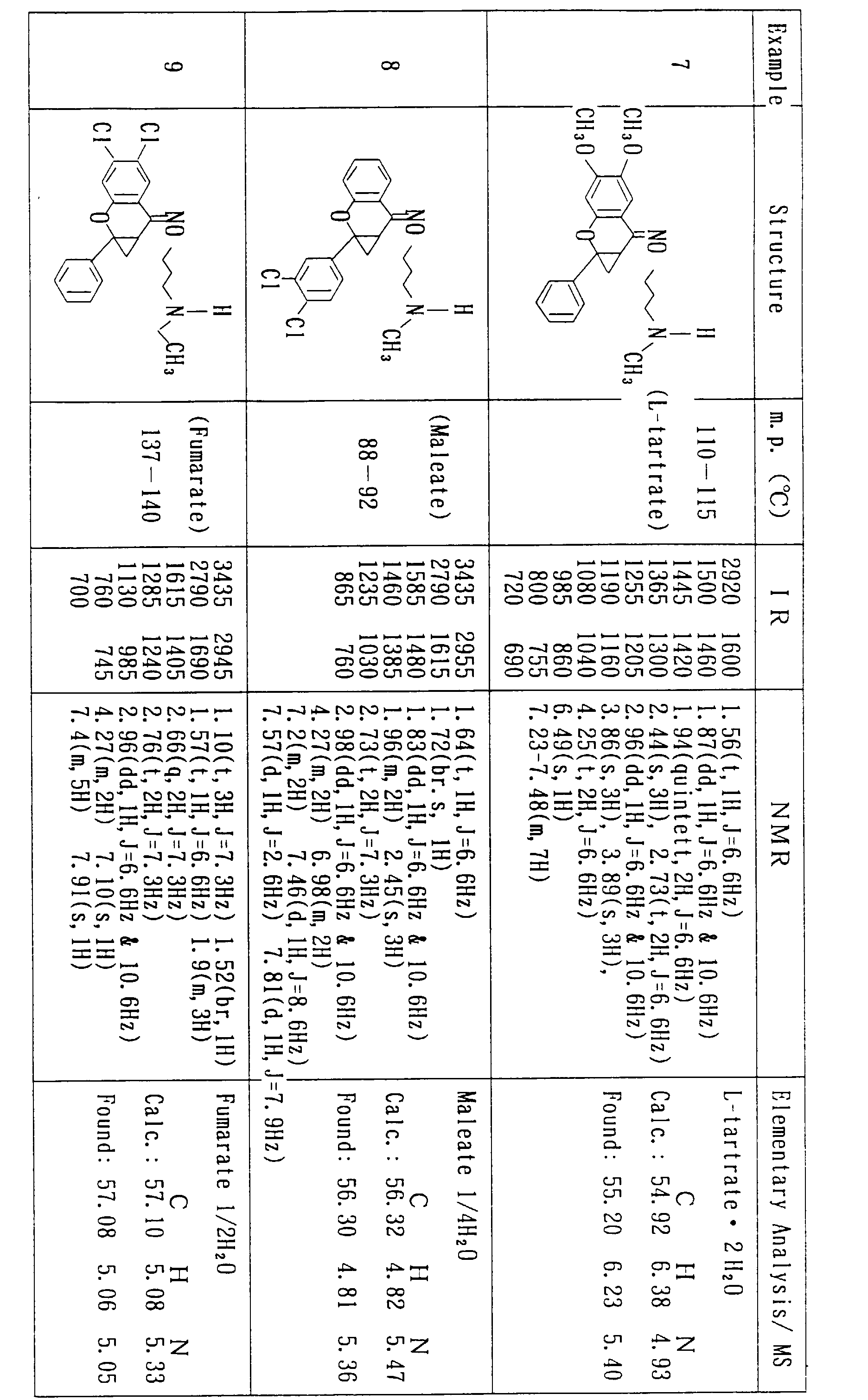

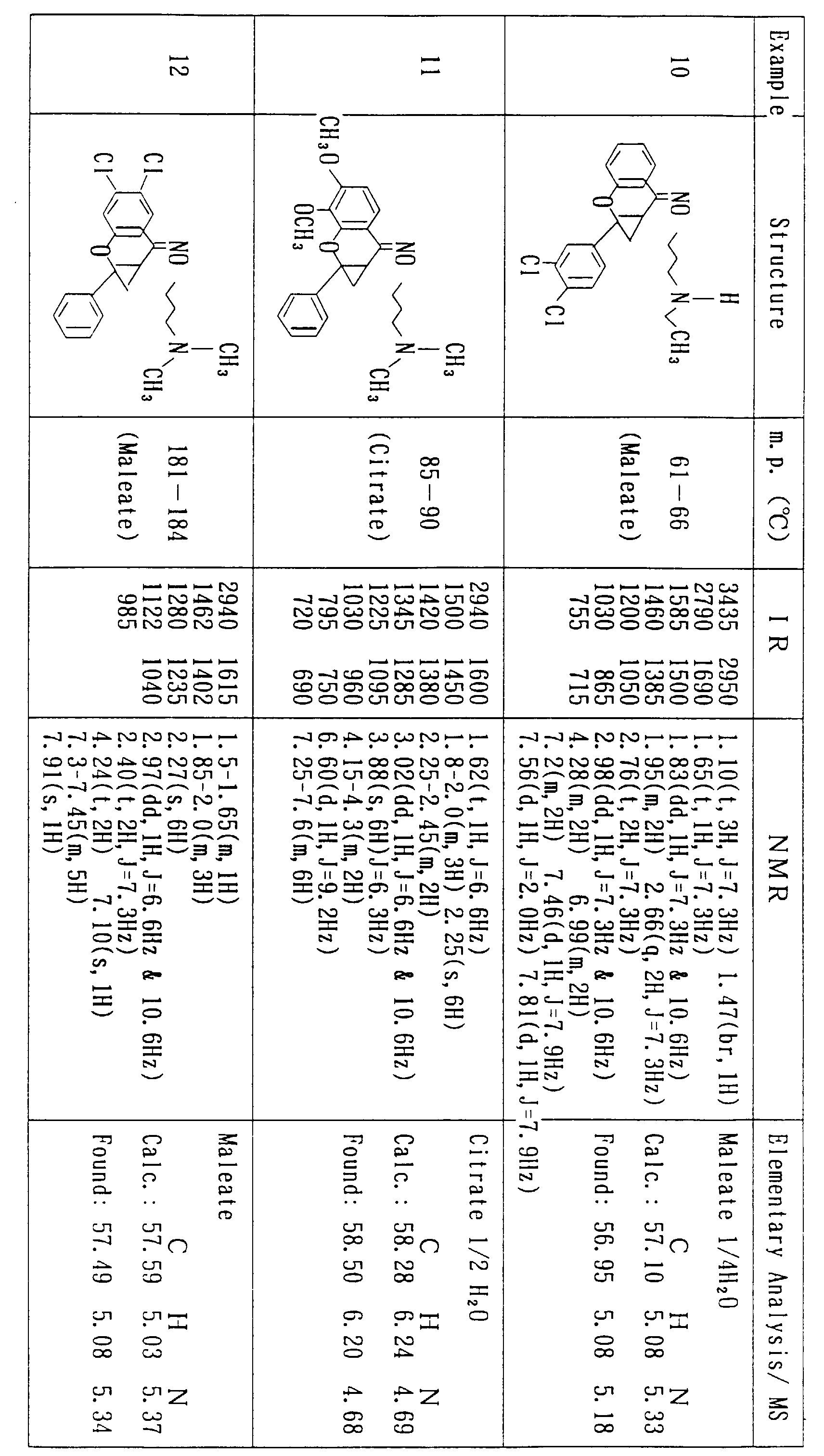

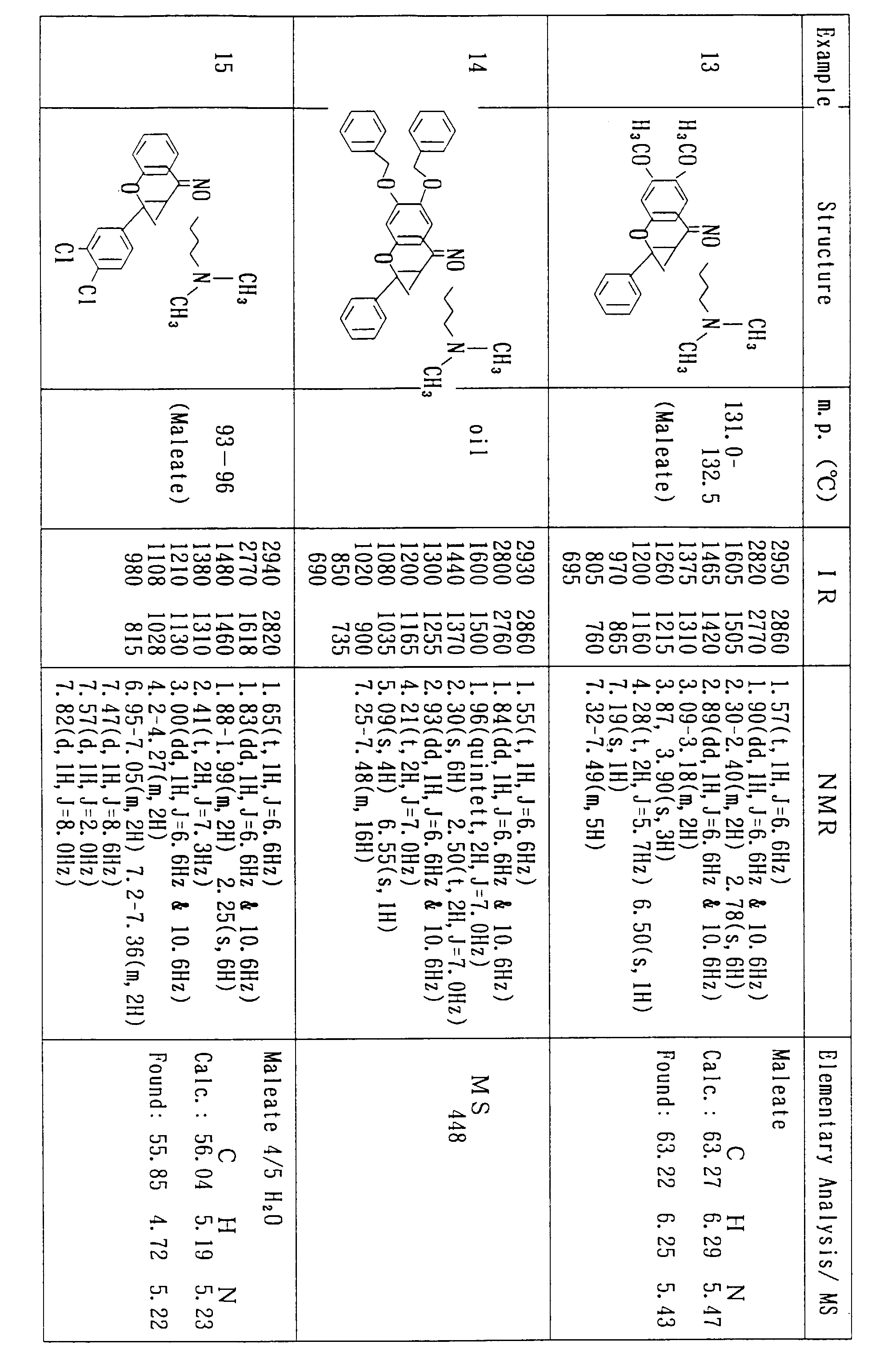

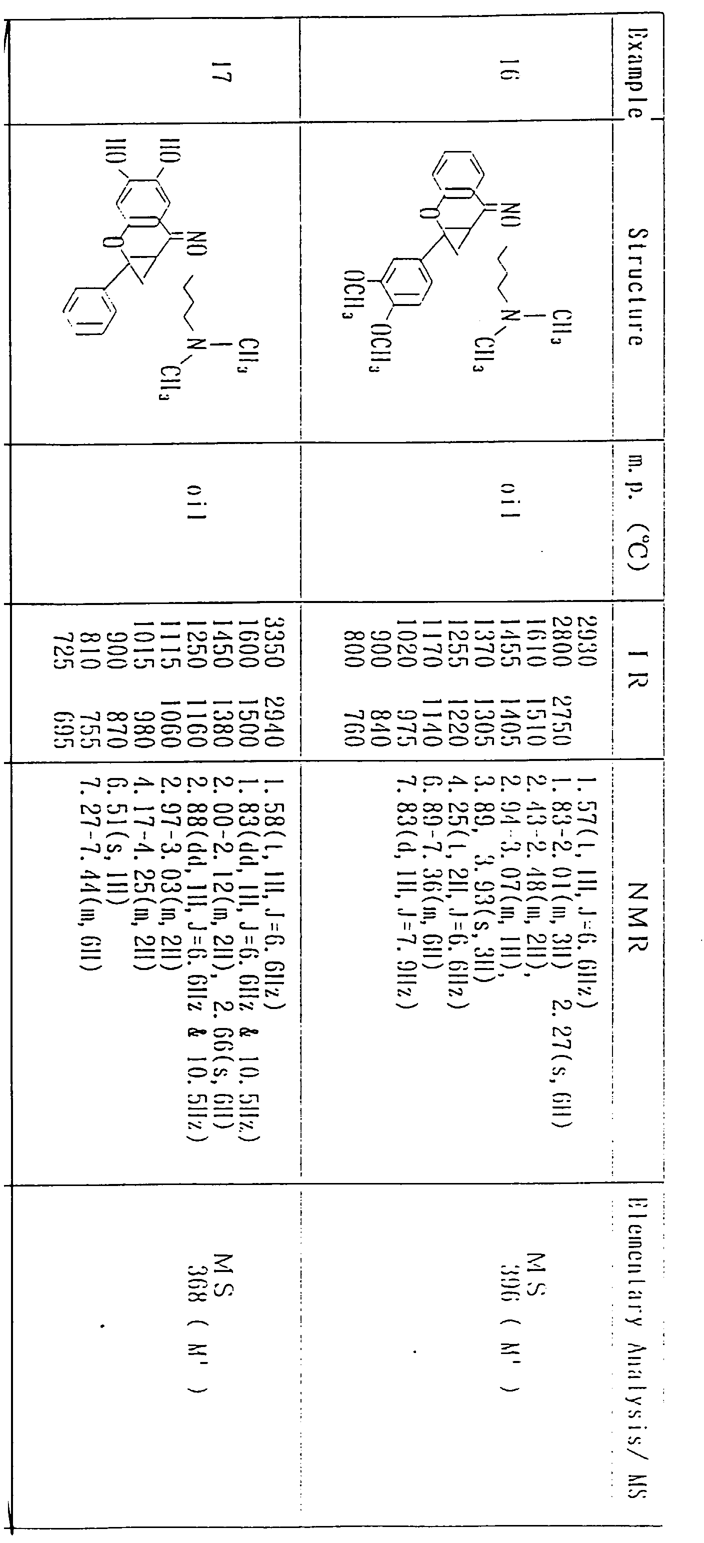

### Test Example

### Antihypoxia Activity (Brain-Protecting Activity against Oxygen Deficit under Reduced Pressure)

Male mice of ddy strain weighing 22 to 30 g (7 to 10 mice per group) were placed in a desiccator having a capacity of about 1 ℓ, and the inner pressure was reduced to 180 mmHg by suction with a vacuum pump. Thirty minutes before the pressure reduction, a test group intraperitoneally received a solution of 25 mg/kg of a test compound, while a control group received only the solvent. A survival time of from the start of pressure reduction up to the respiratory cessation was measured. In cases when an animal survived more than 15 minutes from the hypoxia induction, the survival time was scored to be 15 minutes. As a result, the compound of the present invention significantly extended the survival time over that of the control group.

## Claims (Claims for the following Contracting State(s): AT, BE, CH, DE, DK, FR, GB, IT, LI, LU, NL, SE)

1. A cyclopropachromen derivative represented by formula (I) : 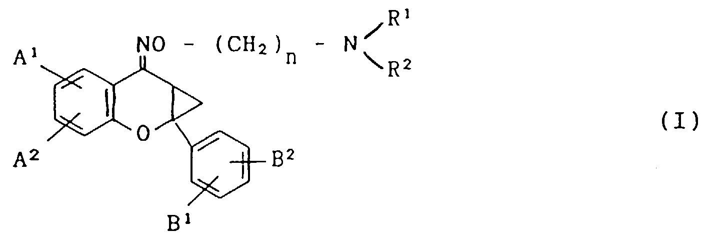 wherein n represents an integer of 2 to 5; R¹ and R² independently represent a hydrogen atom or an alkyl group having from 1 to 5 carbon atoms, a phenyl group or an aralkyl group having 7 to 10 carbon atoms;
A¹ and A² independently represent a hydrogen atom, a hydroxyl group, a halogen atom or a
C₁₋₁₀ alkoxy group optionally substituted by a phenyl group or a pyridyl group; B¹ and B² independently represent a hydrogen atom, a hydroxyl group, a halogen atom or a
C₁₋₁₀ alkoxy group optionally substituted by a phenyl group or a pyridyl group; with the proviso that both substituents or at least one of the benzene rings represent substituents other than hydrogen atoms, and a pharmaceutically acceptable salt thereof.

2. A compound as claimed in Claim 1 wherein one of R¹ and R² represents a hydrogen atom and the other represents an alkyl group having 1 to 5 carbon atoms, a phenyl group or an aralkyl group having 7 to 10 carbon atoms, and A¹ and A² both represent a meaning other than a hydrogen atom.

3. A compound as claimed in Claim 2 wherein A¹ and A² independently represent an alkoxy group having 1 to 5 carbon atoms which may optionally be substituted with a phenyl group, or represent a halogen atom or a hydroxyl group.

4. A compound as claimed in Claim 1 wherein one of R¹ and R² represents a hydrogen atom and the other represents an alkyl group having 1 to 5 carbon atoms, a phenyl group or an aralkyl group having 7 to 10 carbon atoms, and B¹ and B² both represent a meaning other than a hydrogen atom.

5. A compound as claimed in Claim 4 wherein B¹ and B² independently represent an alkoxy group having 1 to 5 carbon atoms or a halogen atom.

6. A compound as claimed in Claim 1 wherein R¹, R², A¹ and A² all represent a meaning other than a hydrogen atom.

7. A compound as claimed in Claim 1 wherein R¹, R², B¹ and B² all represent a meaning other than a hydrogen atom.

8. A cyclopropachromen derivative according to Claims 1 to 7 or its pharmaceutically acceptable salt for use as a medicament.

9. Use of a cyclopropachromen derivative according to Claims 1 to 7 or its pharmaceutically acceptable salt for the manufacture of a medicament for cerebral organic and functional disorders.

## Claims (Claims for the following Contracting State(s): ES, GR)

1. A method for preparing a cyclopropachromen derivative represented by formula (I): 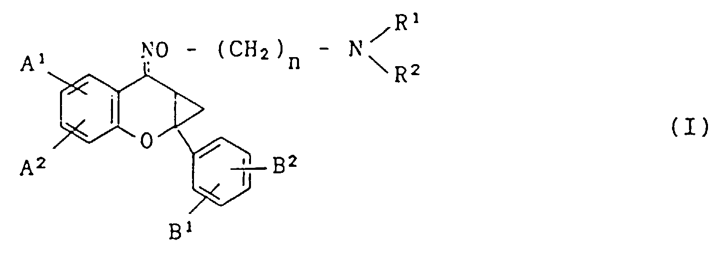 wherein n represents an integer of 2 to 5; R¹ and R² independently represent a hydrogen atom or an alkyl group having from 1 to 5 carbon atoms, a phenyl group or an aralkyl group having 7 to 10 carbon atoms;
A¹ and A² independently represent a hydrogen atom, a hydroxyl group, a halogen atom or a
C₁₋₁₀ alkoxy group optionally substituted by a phenyl group or a pyridyl group; B¹ and B² independently represent a hydrogen atom, a hydroxyl group, a halogen atom or a
C₁₋₁₀ alkoxy group optionally substituted by a phenyl group or a pyridyl group; with the proviso that both substituents or at least one of the benzene rings represent substituents other than hydrogen atoms, and a pharmaceutically acceptable salt thereof, by reacting a compound of formula (II): 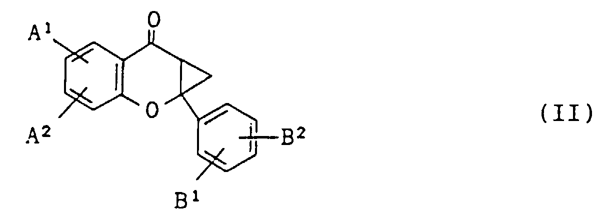 wherein A¹, A², B¹ and B² are defined as above with hydroxylamine hydrochloride in pyridine to obtain compound (III): 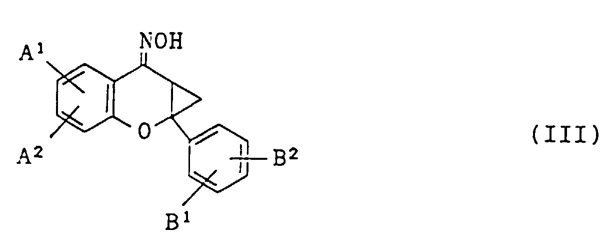 and then condensing compound (III) with a halogenated amine compound of formula 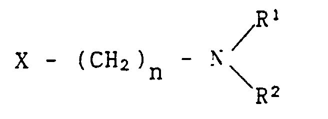 wherein R¹, R² and n are as defined above, and X represents a halogen atom,
to obtain compound (I),
or by condensing compound (III) with a bifunctional compound represented by formula:
X¹ - (CH₂)ₙ - X²
wherein X¹ represents a halogen atom and X² represents a halogen atom or an ethylene oxide group,
to form compound (IV): 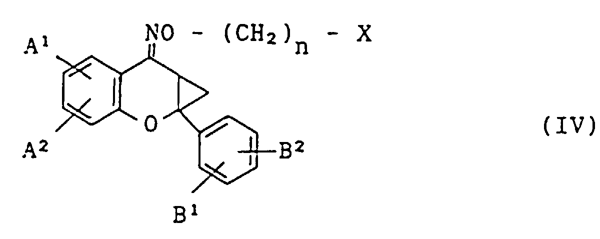
and then reacting compound (IV) with an appropriate amine compound to obtain compound (I) and by optionally converting compound (I) in its pharmaceutically acceptable salt.

2. A method for preparing a cyclopropachromen derivative according to claim 1 wherein one of R¹ and R² represents a hydrogen atom and the other represents an alkyl group having 1 to 5 carbon atoms, a phenyl group or an aralkyl group having 7 to 10 carbon atoms, and A¹ and A² both represent a meaning other than a hydrogen atom.

3. A method for preparing a cyclopropachromen derivative according to claim 2 wherein A¹ and A² independently represent an alkoxy group having 1 to 5 carbon atoms which may optionally be substituted with a phenyl group, or represent a halogen atom or a hydroxyl group.

4. A method for preparing a cyclopropachromen derivative according to claim 1 wherein one of R¹ and R² represents a hydrogen atom and the other represents an alkyl group having 1 to 5 carbon atoms, a phenyl group or an aralkyl group having 7 to 10 carbon atoms, and B¹ and B² both represent a meaning other than a hydrogen atom.

5. A method for preparing a cyclopropachromen derivative according to claim 4 wherein B¹ and B² independently represent an alkoxy group having 1 to 5 carbon atoms or a halogen atom.

6. A method for preparing a cyclopropachromen derivative according to claim 1 wherein R¹, R², A¹ and A² all represent a meaning other than a hydrogen atom.

7. A method for preparing a cyclopropachromen derivative according to claim 1 wherein R¹, R², B¹ and B² all represent a meaning other than a hydrogen atom.

8. Use of a cyclopropachromen derivative as defined in claims 1 to 7 or its pharmaceutically acceptable salt for the manufacture of a medicament.

9. Use of a cyclopropachromen derivative as defined in claims 1 to 7 or its pharmaceutically acceptable salt for the manufacture of a medicament for cerebral and functional disorders.

10. A method for preparing a medicament by mixing a cyclopropachromen derivative as defined in claims 1 to 7 or its pharmaceutically acceptable salt with other ingredients.

11. A method for preparing a medicament for cerebral and functional disorders by mixing a cyclopropachromen derivative as defined in claims 1 to 7 or its pharmaceutically acceptable salt with other ingredients.

## Patentansprüche (Patentansprüche für folgende(n) Vertragsstaat(en): AT, BE, CH, DE, DK, FR, GB, IT, LI, LU, NL, SE)

1. Cyclopropachromenderivat mit der Formel (I) : 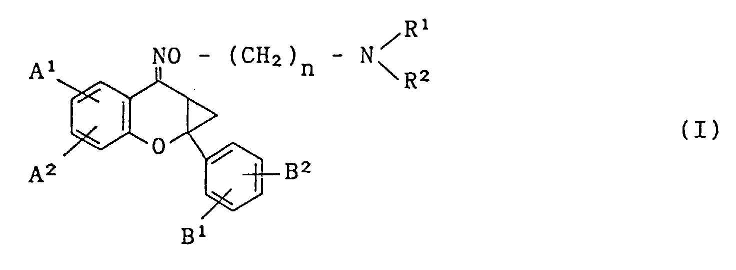 worin n eine ganze Zahl von 2 bis 5 bedeutet; R¹ und R² unabhängig ein Wasserstoffatom oder eine Alkylgruppe mit 1 bis 5 Kohlenstoffatomen, eine Phenylgruppe oder eine Aralkylgruppe mit 7 bis 10 Kohlenstoffatomen sind; A¹ und A² unabhängig ein Wasserstoffatom, eine Hydroxylgruppe, ein Halogenatom oder eine C₁₋₁₀-Alkoxygruppe, die wahlweise mit einer Phenylgruppe oder einer Pyridylgruppe substituiert sein kann, sind; B¹ und B² unabhängig ein Wasserstoffatom, eine Hydroxylgruppe, ein Halogenatom oder eine C₁₋₁₀-Alkoxygruppe, die wahlweise mit einer Phenylgruppe oder Pyrridylgruppe substituiert sein kann, bedeuten, unter der Vorausetzung, daß beide Substituenten an wenigstens einem der Benzolringe von Wasserstoff verschiedene Substituenten bedeuten, und deren pharmazeutisch verträgliche Salze.

2. Verbindung gemäß Anspruch 1, worin einer von R¹ und R² ein Wasserstoffatom ist und der andere eine Alkylgruppe mit 1 bis 5 Kohlenstoffatomen, eine Phenylgruppe oder eine Aralkylgruppe mit 7 bis 10 Kohlenstoffatomen ist, und A¹ und A² beide von Wasserstoff verschiedene Gruppe bedeuten.

3. Verbindung gemäß Anspruch 2, in der A¹ und A² unabhängig voneinander eine Alkoxygruppe mit 1 bis 5 Kohlenstoffatomen, die wahlweise mit einer Phenylgruppe substituiert sein kann, oder ein Halogenatom oder eine Hydroxylgruppe bedeuten.

4. Verbindung gemäß Anspruch 1, in der einer von R¹ und R² ein Wasserstoffatom ist, und der andere eine Alkylgruppe mit 1 bis 5 Kohlenstoffatomen, eine Phenylgruppe oder eine Aralkylgruppe mit 7 bis 10 Kohlenstoffatomen ist, und B¹ und B² beide eine von Wasserstoff verschiedene Gruppe bedeuten.

5. Verbindung gemäß Anspruch 4, in der B¹ und B² unabhängig voneinander eine Alkoxygruppe mit 1 bis 5 Kohlenstoffatomen oder ein Wasserstoffatom bedeuten.

6. Verbindung gemäß Anspruch 1, in der R¹, R², A¹ und A² alle von Wasserstoff verschiedene Gruppen bedeuten.

7. Verbindung gemäß Anspruch 1, in der R¹, R², B¹ und B² alle von Wasserstoff verschiedene Gruppen bedeuten.

8. Ein Cyclopropachromenderivat gemäß Anspruch 1 bis 7 oder deren pharmazeutisch verträgliche Salze zur Verwendung als Medikament.

9. Verwendung eines Cyclopropachromenderivats gemäß Ansprüchen 1 bis 7, oder deren pharmazeutisch verträgliche Salze zur Herstellung eines Medikaments gegen zerebrale organische und funktionale Störungen.

## Patentansprüche (Patentansprüche für folgende(n) Vertragsstaat(en): ES, GR)

1. Verfahren zur Herstellung eines Cyclopropachromenderivat mit der Formel (I): 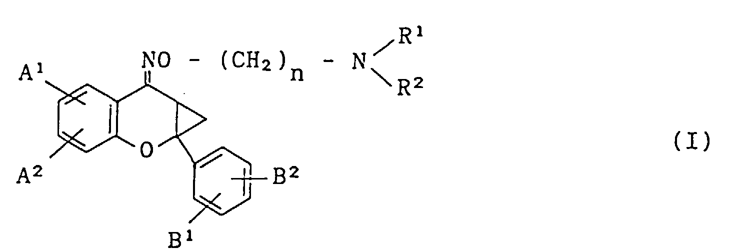 worin n eine ganze Zahl von 2 bis 5 bedeutet; R¹ und R² unabhängig ein Wasserstoffatom oder eine Alkylgruppe mit 1 bis 5 Kohlenstoffatomen, eine Phenylgruppe oder eine Aralkylgruppe mit 7 bis 10 Kohlenstoffatomen sind; A¹ und A² unabhängig ein Wasserstoffatom, eine Hydroxylgruppe, ein Halogenatom oder eine C₁₋₁₀-Alkoxygruppe, die wahlweise mit einer Phenylgruppe oder einer Pyridylgruppe substituiert sein kann, sind; B¹ und B² unabhängig ein Wasserstoffatom, eine Hydroxylgruppe, ein Halogenatom oder eine C₁₋₁₀-Alkoxygruppe, die wahlweise mit einer Phenylgruppe oder Pyridylgruppe substituiert sein kann, bedeuten, unter der Vorausetzung, daß beide Substituenten oder wenigstens einer der Benzolringe von Wasserstoff verschiedene Substituenten bedeuten, und ein pharmazeutisch verträgliches Salz davon, durch Umsetzen einer Verbindung mit der Formel (II): 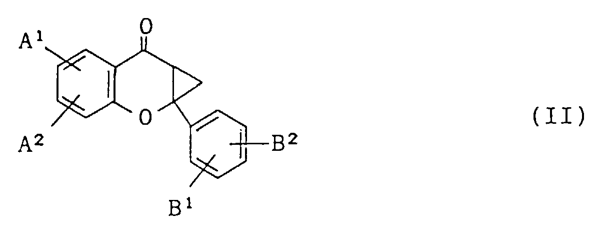 worin A¹, A², B¹ und B² wie oben definiert sind mit Hydroxylaminhydrochlorid in Pyridin, um Verbindung (III) zu erhalten: 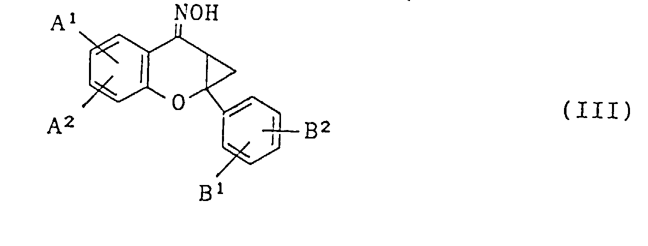 und anschließendes Kondensieren von Verbindung (III) mit einer halogenierten Aminverbindung mit der Formel 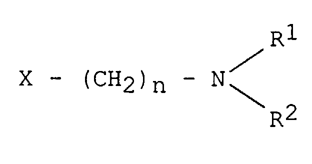 worin R¹, R² und n wie oben definiert sind, und X ein Halogenatom ist,
um Verbindung (I) zu erhalten,
oder durch Kondensieren von Verbindung (III) mit einer bifunktionalen Verbindung mit der Formel:
X¹ - (CH₂)ₙ - X²
worin X¹ ein Halogenatom und X² ein Halogenatom oder eine Ethylenoxidgruppe sind,
um Verbindung (IV) zu bilden: 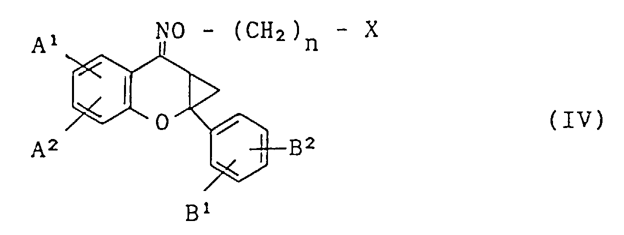 und dann Umsetzen von Verbindung (IV) mit einer entsprechenden Aminverbindung, um Verbindung (I) zu erhalten und wahlweises Überführen der Verbindung (I) in deren pharmazeutisch verträgliche Salze.

2. Verfahren zur Herstellung eines Cyclopropachromenderivats gemäß Anspruch 1, worin einer von R¹ und R² ein Wasserstoffatom ist und der andere eine Alkylgruppe mit 1 bis 5 Kohlenstoffatomen, eine Phenylgruppe oder eine Aralkylgruppe mit 7 bis 10 Kohlenstoffatomen ist, und A¹ und A² beide von Wasserstoff verschiedene Gruppe bedeuten.

3. Verfahren zur Herstellung eines Cyclopropachromenderivats gemäß Anspruch 2, in der A¹ und A² unabhängig voneinander eine Alkoxygruppe mit 1 bis 5 Kohlenstoffatomen, die wahlweise mit einer Phenylgruppe substituiert sein kann, oder ein Halogenatom oder eine Hydroxylgruppe bedeuten.

4. Verfahren zur Herstellung eines Cyclopropachromenderivats gemäß Anspruch 1, in der einer von R¹ und R² ein Wasserstoffatom ist, und der andere eine Alkylgruppe mit 1 bis 5 Kohlenstoffatomen, eine Phenylgruppe oder eine Aralkylgruppe mit 7 bis 10 Kohlenstoffatomen ist, und B¹ und B² beide eine von Wasserstoff verschiedene Gruppe bedeuten.

5. Verfahren zur Herstellung eines Cyclopropachromenderivats gemäß Anspruch 4, in der B¹ und B² unabhängig voneinander eine Alkoxygruppe mit 1 bis 5 Kohlenstoffatomen oder ein Wasserstoffatom bedeuten.

6. Verfahren zur Herstellung eines Cyclopropachromenderivats gemäß Anspruch 1, in der R¹, R², A¹ und A² alle von Wasserstoff verschiedene Gruppen bedeuten.

7. Verfahren zur Herstellung eines Cyclopropachromenderivats gemäß Anspruch 1, in der R¹, R², B¹ und B² alle von Wasserstoff verschiedene Gruppen bedeuten.

8. Verwendung eines Cyclopropachromenderivats, wie in Ansprüchen 1 bis 7 definiert, oder dessen pharmazeutisch verträgliche Salze zur Herstellung eines Medikaments.

9. Verwendung eines Cyclopropachromenderivats, wie in Ansprüchen 1 bis 7 definiert, oder dessen pharmazeutisch verträgliche Salze zur Herstellung eines Medikaments gegen zerebrale und funktionale Störungen.

10. Verfahren zur Herstellung eines Medikaments durch Mischen eines Cyclopropachromenderivats, wie in Ansprüchen 1 bis 7 definiert, oder dessen pharmazeutisch verträglicher Salze, mit anderen Zutaten.

11. Verfahren zur Herstellung eines Medikaments gegen zerebrale und funktionale Störungen durch Mischen eines Cyclopropachromenderivats, wie in Ansprüchen 1 bis 7 definiert, oder dessen pharmazeutisch verträglicher Salze, mit anderen Zutaten.

## Revendications (Revendications pour l'(les) Etat(s) contractant(s) suivant(s): AT, BE, CH, DE, DK, FR, GB, IT, LI, LU, NL, SE)

1. Un dérivé de cyclopropachromène représenté par la formule (I) : 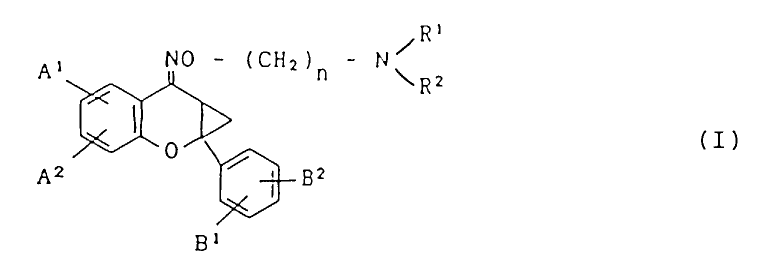 dans laquelle n représente un nombre entier de 2 à 5 ; R¹ et R² représentent chacun indépendamment un atome d'hydrogène ou un groupe alkyle ayant 1 à 5 atomes de carbone, un groupe phényle ou un groupe aralkyle ayant 7 à 10 atomes de carbone ; A¹ et A² représentent chacun indépendamment un atome d'hydrogène, un groupe hydroxyle, un atome d'halogène ou un groupe alcoxy en C₁-C₁₀ facultativement substitué par un groupe phényle ou un groupe pyridyle ; B¹ et B² représentent chacun indépendamment un atome d'hydrogène, un groupe hydroxyle, un atome d'halogène ou un groupe alcoxy en C₁-C₁₀ facultativement substitué par un groupe phényle ou un groupe pyridyle ; à condition que les deux substituants sur au moins l'un des noyaux benzéniques ne soient pas des atomes d'hydrogène, et un sel pharmaceutiquement acceptable de ce dérivé.

2. Un composé tel que revendiqué dans la revendication 1, dans lequel l'un de R¹ et R² représente un atome d'hydrogène et l'autre représente un groupe alkyle ayant 1 à 5 atomes de carbone, un groupe phényle ou un groupe aralkyle ayant 7 à 10 atomes de carbone, et chacun de A¹ et A² ne soit pas un atome d'hydrogène.

3. Un composé tel que revendiqué dans la revendication 2, dans lequel chacun de A¹ et A², indépendamment, représente un groupe alcoxy ayant 1 à 5 atomes de carbone qui peut être facultativement substitué par un groupe phényle, ou représente un atome d'halogène ou un groupe hydroxyle.

4. Un composé tel que revendiqué dans la revendication 1, dans lequel l'un de R¹ et R² représente un atome d'hydrogène et l'autre représente un groupe alkyle ayant 1 à 5 atomes de carbone, un groupe phényle ou un groupe aralkyle ayant 7 à 10 atomes de carbone, et chacun de B¹ et B² ne qoit pas un atome d'hydrogène.

5. Un composé tel que revendiqué dans la revendication 4, dans lequel B¹ et B² représentent chacun indépendamment un groupe alcoxy ayant 1 à 5 atomes de carbone ou un atome d'halogène.

6. Un composé tel que revendiqué dans la revendication 1, dans lequel R¹, R², A¹ et A² ne représentent pas un atome d'hydrogène.

7. Un composé tel que revendiqué dans la revendication 1, dans lequel R¹, R², B¹ et B² ne représentent pas un atome d'hydrogène.

8. Un dérivé de cyclopropachromène selon les revendications 1 à 7 ou son sel pharmaceutiquement acceptable, pour son utilisation comme médicament.

9. Utilisation d'un dérivé de cyclopropachromène selon les revendications 1 à 7 ou de son sel pharmaceutiquement acceptable pour la fabrication d'un médicament destiné à des troubles cérébraux organiques et fonctionnels.

## Revendications (Revendications pour l'(les) Etat(s) contractant(s) suivant(s): ES, GR)

1. Un procédé pour préparer un dérivé de cyclopropachromène représenté par la formule (I) : 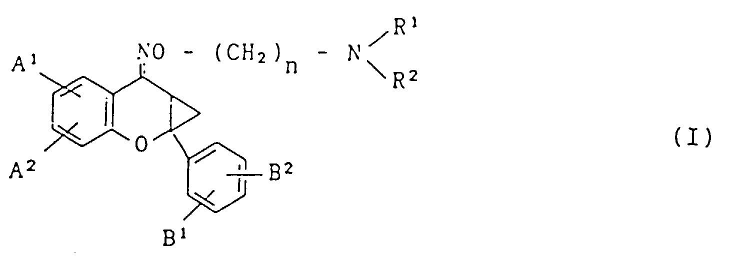 dans laquelle n représente un nombre entier de 2 à 5 ; R¹ et R² représentent chacun indépendamment un atome d'hydrogène ou un groupe alkyle ayant 1 à 5 atomes de carbone, un groupe phényle ou un groupe aralkyle ayant 7 à 10 atomes de carbone ; A¹ et A² représentent chacun indépendamment un atome d'hydrogène, un groupe hydroxyle, un atome d'halogène ou un groupe alcoxy en C₁-C₁₀ facultativement substitué par un groupe phényle ou un groupe pyridyle ; B¹ et B² représentent chacun indépendamment un atome d'hydrogène, un groupe hydroxyle, un atome d'halogène ou un groupe alcoxy en C₁-C₁₀ facultativement substitué par un groupe phényle ou un groupe pyridyle ; à condition que les deux substituants sur au moins l'un des noyaux benzéniques en soient pas des atomes d'hydrogène, et d'un sel pharmaceutiquement acceptable de ce dérivé, par réaction d'un composé de formule (II) : 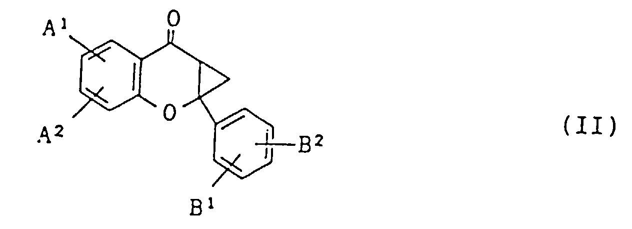 ou A¹, A², B¹ et B² sont tels que définis ci-dessus, avec le chlorhydrate d'hydroxylamine dans la pyridine pour donner le composé (III) : 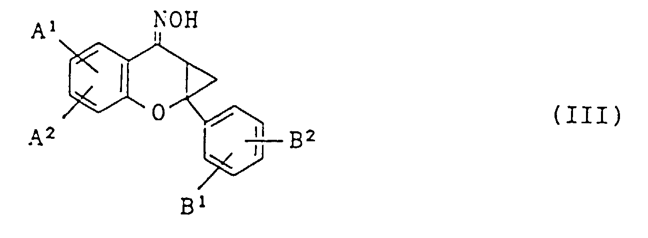 puis condensation du composé (III) avec une amine halogénée de formule : 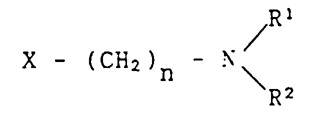 où R¹, R² et n sont tels que définis ci-dessus et X représente un atome d'halogène, pour produire le composé (I), ou par condensation du composé (III) avec un composé difonctionnel représenté par la formule :
X¹ - (CH₂)ₙ - X²
où X¹ représente un atome d'halogène et X² représente un atome d'halogène ou un groupe oxyde d'éthylène, pour former le composé (IV) : 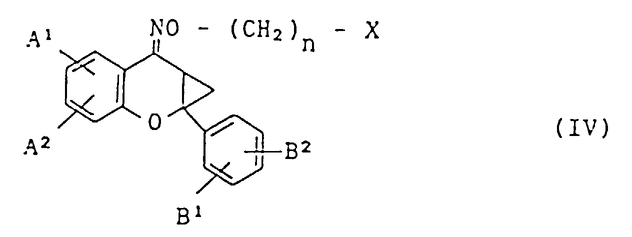 puis réaction du composé (IV) avec une amine appropriée pour produire le composé (I), et conversion facultative du composé (I) en son sel pharmaceutiquement acceptable.

2. Un procédé pour préparer un dérivé de cyclopropachromène selon la revendication 1, dans lequel l'un de R¹ et R² représente un atome d'hydrogène et l'autre représente un groupe alkyle ayant 1 à 5 atomes de carbone, un groupe phényle ou un groupe aralkyle ayant 7 à 10 atomes de carbone, et chacun de A¹ et A² ne représente pas un atome d'hydrogène.

3. Un procédé pour préparer un dérivé de cyclopropachromène selon la revendication 2, dans lequel chacun de A¹ et A², indépendamment, représente un groupe alcoxy ayant 1 à 5 atomes de carbone qui peut être facultativement substitué par un groupe phényle, ou représente un atome d'halogène ou un groupe hydroxyle.

4. Un procédé pour préparer un dérivé de cyclopropachromène selon la revendication 1, dans lequel l'un de R¹ et R² représente un atome d'hydrogène et l'autre représente un groupe alkyle ayant 1 à 5 atomes de carbone, un groupe phényle ou un groupe aralkyle ayant 7 à 10 atomes de carbone, et chacun de B¹ et B² ne représente pas un atome d'hydrogène.

5. Un procédé pour préparer un dérivé de cyclopropachromène selon la revendication 4, dans lequel B¹ et B² représentent chacun indépendamment un groupe alcoxy ayant 1 à 5 atomes de carbone ou un atome d'halogène.

6. Un procédé pour préparer un dérivé de cyclopropachromène selon la revendication 1, dans lequel R¹, R², A¹ et A² ne représentent pas un atome d'hydrogène.

7. Un procédé pour préparer un dérivé de cyclopropachromène selon la revendication 1, dans lequel R¹, R², B¹ et B² ne représentent pas un atome d'hydrogène.

8. Utilisation d'un dérivé de cyclopropachromène tel que défini dans les revendications 1 à 7 ou de son sel pharmaceutiquement acceptable pour la fabrication d'un médicament.

9. Utilisation d'un dérivé de cyclopropachromène tel que défini dans les revendications 1 à 7 ou de son sel pharmaceutiquement acceptable pour la fabrication d'un médicament destiné à des troubles cérébraux et fonctionnels.

10. Un procédé pour préparer un médicament, par mélange d'un dérivé de cyclopropachromène tel que défini dans les revendications 1 à 7 ou de son sel pharmaceutiquement acceptable avec d'autres ingrédients.

11. Un procédé pour préparer un médicament destiné à des troubles cérébraux et fonctionnels, par mélange d'un dérivé de cyclopropachromène tel que défini dans les revendications 1 à 7 ou de son sel pharmaceutiquement acceptable avec d'autres ingrédients.
